(19) Europäisches Patentamt
European Patent Office
Office européen des brevets

(11) **EP 3 111 207 B1**

(12) **EUROPÄISCHE PATENTSCHRIFT**

(45) Veröffentlichungstag und Bekanntmachung des Hinweises auf die Patenterteilung:
**06.06.2018 Patentblatt 2018/23**

(21) Anmeldenummer: **15778196.4**

(22) Anmeldetag: **31.07.2015**

(51) Int Cl.:
*G01N 29/024* (2006.01)    *A61B 5/08* (2006.01)
*G01N 29/22* (2006.01)    *G01N 33/00* (2006.01)

(86) Internationale Anmeldenummer:
**PCT/DE2015/100323**

(87) Internationale Veröffentlichungsnummer:
**WO 2016/019945 (11.02.2016 Gazette 2016/06)**

(54) **VERFAHREN UND VORRICHTUNG ZUR BESTIMMUNG DES ANTEILS AN MOLEKULAREM SAUERSTOFF IN EINEM ATEMGAS MITTELS SCHALL**

METHOD AND APPARATUS FOR DETERMINATION OF THE MOLECULAR OXYGEN FRACTION IN BREATHING GAS BY MEANS OF SOUND

PROCÉDÉ ET DISPOSITIF POUR LA DÉTERMINATION DU FRACTION D'OXYGÈNE MOLÉCULAIRE DANS UN GAZ DE RESPIRATION PAR DU SON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.08.2014 DE 102014111366**

(43) Veröffentlichungstag der Anmeldung:
**04.01.2017 Patentblatt 2017/01**

(73) Patentinhaber: **Ganshorn, Peter**
**97702 Münnerstadt (DE)**

(72) Erfinder: **Ganshorn, Peter**
**97702 Münnerstadt (DE)**

(74) Vertreter: **Pöhner, Wilfried Anton**
**Patentanwalt Dr. W. Pöhner**
**Kaiserstrasse 33**
**97070 Würzburg (DE)**

(56) Entgegenhaltungen:
**WO-A1-2013/088289     DE-A1- 4 318 690**
**DE-A1-102008 056 279**

**Beschreibung**

Die Erfindung betrifft ein Verfahren zur Bestimmung des Anteils an molekularem Sauerstoff in einem Atemgas, beispielsweise in der Lungenfunktionsdiagnostik, umfassend das Einleiten des Atemgases in ein Messrohr, das Senden eines Schallsignals durch einen Schallsender und Empfangen des Schallsignals durch einen Schallempfänger, das Definieren einer Schallmessstrecke durch den Schallsender und den Schallempfänger, das Bestimmen der mittleren molaren Masse des Atemgases mittels der über die Schallmessstrecke gemessenen Schalllaufzeit, und das Bestimmen des Kohlenstoffdioxidanteils des Atemgases mit einem Kohlenstoffdioxidgassensor. Zudem betrifft die Erfindung eine Vorrichtung zur Durchführung des Verfahrens.

In einer Vielzahl medizinischer Anwendungen ist die Bestimmung des Anteils an molekularem Sauerstoff in Atemgasen unerlässlich, wie beispielsweise in der Notfall- und Intensivmedizin, der Anästhesie und der Lungenfunktionsdiagnostik. Mittels des ermittelten Sauerstoffanteils lassen sich Rückschlüsse auf u.a. die Lungenfunktionalität, den respiratorischen Quotienten und die Stoffwechselrate des Probanden ziehen.

Herkömmliche Systeme zur Untersuchung von Atemgasen enthalten eine Vielzahl unterschiedlicher Sensoren und basieren auf der Messung diverser Atemgas-Parameter wie Strömungsgeschwindigkeit, Temperatur, Feuchtigkeit, Druck und der konkreten Atemgaszusammensetzung. Die Bestimmung der Zusammensetzung des Atemgases erfolgt vorwiegend im Neben- oder Hauptstrom anhand unterschiedlicher, teils aufwendiger und teurer Messverfahren, wie beispielsweise der Infrarotspektroskopie, dem Paramagnetismus, mittels Laser oder der Massenspektroskopie.

Atemgas besteht typischerweise aus den im Folgenden genannten Komponenten bzw. Elementen: Stickstoff ($N_2$), Sauerstoff ($O_2$), Argon (Ar), Wasserdampf ($H_2O$) und diversen Spurengasen, wie Kohlenstoffdioxid ($CO_2$), Ozon, Kohlenstoffmonoxid und verschiedenen Edelgasen. Die mittlere Molmasse des Atemgases ergibt sich aus der Summe der Produkte der Molmassen und Stoffmengenanteile der einzelnen Komponenten oder Elemente, hauptsächlich jedoch aus der Summe der Produkte der Molmassen von Sauerstoff, Stickstoff, Kohlenstoffdioxid und Argon.

Die mittlere molare Masse des Atemgases ist allerdings auch abhängig von dessen Feuchtigkeit und damit von Druck und Temperatur. Enthält das Atemgas beispielsweise Feuchtigkeit, verringert sich dessen mittlere Molmasse, da die Molmasse von Wasserdampf kleiner ist als die mittlere Molmasse trockenen Atemgases. Diese Abhängigkeit der mittleren molaren Masse des Atemgases führt dazu, dass für die Bestimmung der Zusammensetzung des Atemgases teils aufwendige und teure Messverfahren nebst aufwendigen Berechnungen notwendig sind.

Moderne Verfahren und Vorrichtungen basieren darauf, die mittlere molare Masse eines Gasgemisches mittels Ultraschallmessung zu bestimmen. Ein derartiges Verfahren ist beispielsweise der Offenlegungsschrift EP 0 533 980 A1 zu entnehmen, in welchem die Konzentration von Kraftstoffen oder Gasen in der Ansaugluft von Kfz-Motoren bestimmt wird.

Für medizinische Anwendungen sind bereits Vorrichtungen zur Messung der Molmasse von Gasgemischen mittels Ultraschall-Laufzeitmessung bekannt. Beispielsweise beschreibt die Offenlegungsschrift EP 1 279368 A2 eine Vorrichtung zur Messung der Strömungsgeschwindigkeit und/oder der Molmasse von Gasen- oder Gasgemischen in medizinischer Anwendung mittels Ultraschall-Laufzeitmessung.

Die Offenlegungsschrift EP 0 646 346 A2 beschreibt eine Vorrichtung zur Messung von Atemgasparametern mit einem Atemrohr, Ultraschallsensoren und einer in einem separaten Gehäuse angeordneten Vorverstärkerelektronik.

Mittels Ultraschallmessung kann die Dichte des Atemgases gemessen werden, um aus dieser die mittlere molare Masse des Atemgases zu bestimmen sind allerdings zusätzliche Informationen erforderlich, wie beispielsweise dessen Temperatur, dessen Feuchtigkeit, dessen Druck und/oder dessen Geschwindigkeit.

Mit den oben beschriebenen Verfahren lassen sich jedoch keine direkten Rückschlüsse aus der mittleren molaren Masse des Atemgases auf dessen konkrete Zusammensetzung bzw. die Konzentration der einzelnen Komponenten oder Elemente schließen, wie beispielsweise auf den Anteil an molekularem Sauerstoff im Atemgas.

In der Patentschrift EP 0 653 919 B1 wird ebenfalls ein Verfahren zur Messung der Molmasse von Gasen oder Gasgemischen mittels Ultraschallmessung und eine Vorrichtung zum Durchführen dieses Verfahrens beschrieben. Zusätzlich wird offenbart, dass sich durch die Kombination der Ultraschallmessung mit einem weiteren Gassensor die Sauerstoffaufnahme, die Kohlenstoffdioxidabgabe und der respispiratorische Quotient bestimmen lässt. Die Patentschrift beschreibt zudem einen Kohlenstoffdioxidgassensor auf Infrarotbasis.

Gleiches gilt für das in der Offenlegungsschrift DE 43 18 690 A1 beschriebene Verfahren zur Messung der Molmasse von Gasen oder Gasgemischen. Durch Ultraschallmessung erfolgt eine Bestimmung der mittleren molaren Masse. Die Anteile bestimmter, interessierender Gase wie Sauerstoff oder Kohlendioxid werden der dortigen Lehre gemäß durch dedizierte Sensoren gemessen, was die Komplexität der für die Durchführung des dortigen Verfahrens benötigten Vorrichtungen nachteilig erhöht. Im Übrigen verläuft die dortige Ultraschallmessstrecke nur teilweise in der Strömung des Atemgases und ansonsten in nur einseitig zur Strömung offenen Kammern, wodurch die Genauigkeit und Geschwindigkeit der Bestimmung der molaren Masse reduziert sind.

Die Druckschrift DE 10 2008 056 279 A1 offenbart eine Vorrichtung, welche in der Lage ist neben einer Ultraschallbestimmung der mittleren molaren Masse auch den Anteil an Kohlendioxid in einem Gasgemisch dadurch zu bestimmen, dass der Sauerstoffanteil explizit gemessen und, gewichtet mit der Molmasse von Sauerstoff, von der mittleren Molaren Masse subtrahiert wird.

Nachteilig ist hierbei die Verwendung eines expliziten Feststoffelektrolyt-Sauerstoffsensors, der eine große Bauform und hohen Energieverbrauch aufgrund der hohen benötigten Betriebstemperatur hat.

Die bisher verfügbaren Systems sind zwar in der Lage, eine Vielzahl verschiedener Atemgasparameter zu bestimmen. Allerding geschieht dies mit einem vergleichsweise hohen technischen Aufwand dieser teils komplex ausgestalteten und damit störanfälligen Verfahren und auch Vorrichtungen. Für die meisten medizinischen Systeme ist allerdings nur der Anteil an molekularem Sauerstoff und Kohlenstoffdioxid im Atemgas von zentraler Bedeutung. Es besteht daher ein großer Bedarf an einem Verfahren, welches auf einfache und verlässliche Weise lediglich den Anteil an molekularem Sauerstoff in Atemgasen bestimmt.

Aufgabe der Erfindung ist es also, ein einfaches und zuverlässig arbeitendes Verfahren zur Bestimmung des Anteils an molekularem Sauerstoff in Atemgasen bereitzustellen, welches in kürzester Zeit die Bestimmung durchführt, um die oben genannten Schwierigkeiten zu überwinden. Eine weitere Aufgabe der Erfindung besteht darin, eine kleine und baulich kompakte Vorrichtung für die Durchführung des Verfahrens zu erhalten.

Diese Aufgabe wird durch das Verfahren mit den Merkmalen des unabhängigen Anspruchs 1 insbesondere dadurch gelöst, dass die Bestimmung des Anteils an molekularem Sauerstoff in dem Atemgas mittels Differenzbildung aus der bestimmten mittleren molaren Masse des Atemgases und aus dem bestimmten Kohlenstoffdioxidanteil erfolgt. Vorteilhafte Weiterbildungen der Erfindung, welche einzeln oder in Kombination realisierbar sind, sind in den Unteransprüchen dargestellt.

Der Begriff "Atemgas" bezeichnet jedes Gasgemisch, das für die Atmung verwendet werden kann, wie beispielsweise Umgebungsluft oder verschiedene Gasgemische, welche in Atemgeräten zur Verwendung kommen.

Der Begriff "Messrohr" ist dem Fachmann bekannt und betrifft jeden Hohlkörper, welcher dazu geeignet ist, dass Atemgas eingeleitet werden kann. Das Messrohr ist eine Bohrung in einem Block bestehend aus einem beliebigen Material, vorzugsweise aus Kunststoff, Metall oder einem keramischen Werkstoff, wie beispielsweise ein Metallblock, oder ist ein Rohr bestehend aus einem beliebigen Material, vorzugsweise aus Kunststoff, Metall oder einem keramischen Werkstoff. Bevorzugt ist das verwendete Messrohr leicht zu reinigen und/oder weist eine glatte Oberfläche auf, d.h. das verwendete Material ist zumindest säure- und/oder laugenbeständig. Alternativ ist das Messrohr gasdicht. Das Messrohr umfasst einen Schallsender und einen Schallempfänger als Schallmessstrecke und einen Kohlenstoffdioxidgassensor. Zusätzlich kann das Messrohr zur Erhöhung der Genauigkeit der Bestimmung des Anteils an molekularem Sauerstoff eine Einrichtung zur Messung der Temperatur, des Luftdruckes und/oder der Feuchtigkeit des Atemgases umfassen.

Der Begriff "Schallsender" ist bekannt und ist eine Quelle oder Einrichtung zur Erzeugung und Abstrahlung von Schallwellen. Entsprechend dem Frequenzbereich unterscheidet ein Fachmann Infraschall (< 16 Hz), Hörschall (16 Hz bis 20 kHz), Ultraschall (20 kHz bis 1,6 GHz) und Hyperschall (> 1 GHz). Bevorzugt wird Ultraschall verwendet, so dass der Schallsender wenigstens eine Ultraschallquelle ist.

Der Begriff "Schallempfänger" ist bekannt und ist ein akustischer Sensor oder ein Mikrofon, welcher Luftschall als Schallwechseldruckschwingungen in entsprechende Spannungsänderungen, vorzugsweise in elektrische Spannungsänderungen, umwandelt. Bevorzugt wird Ultraschall verwendet, so dass der Schallempfänger wenigstens ein akustischer Sensor oder ein Mikrofon ist. Einem Fachmann sind der Aufbau und die Funktionsweise eines Schallsenders und eines Schallempfängers bekannt.

Bevorzugt wird in einer Ausgestaltung der vorliegenden Erfindung Ultraschall verwendet. In einem solchen Fall bieten sich als Schallsender und Schallempfänger Piezoschwinger an. Noch mehr bevorzugt ist ein- und derselbe Piezoschwinger sowohl für die Bestimmung der mittleren molaren Masse des Atemgases im Wechsel als Schallsender und als Schallempfänger zu betreiben.

Bevorzugt sind der Schallsender und der Schallempfänger an bzw. in das Messrohr unlösbar integriert, so dass diese mit dem Messrohr ein einstückiges Teil bilden. Diese sind dann beispielsweise in entsprechende Ansätze des Messrohres eingegossen. Auf diese Weise lässt sich das Messrohr mit dem Schallsender und dem Schallempfänger problemlos austauschen. Bevorzugt sind der Schallsender und der Schallempfänger an bzw. mit dem Messrohr lösbar verbunden, so dass diese und das Messrohr leicht voneinander getrennt werden können. Auf diese Weise lassen sich das Messrohr und der Schallsender und Schallempfänger unabhängig voneinander austauschen.

Weiterhin bevorzugt umfasst der Schallsender und/oder der Schallempfänger eine Vorverstärkerelektronik, welche unlösbar in bzw. an dem Messrohr integriert oder lösbar mit diesem verbunden ist. Ein Fachmann kennt verschiedene Möglichkeiten, um dies zu realisieren.

Der Schallsender und der Schallempfänger definieren eine Schallmessstrecke von bekannter Länge, welche bevorzugt im bzw. durch das Messrohr verläuft. Der Schallsender und der Schallempfänger können dabei jede Position zueinander einnehmen, beispielsweise gegenüberliegend und/oder nebeneinander. Es versteht sich, dass, beispielsweise im Fall der Anordnung nebeneinanderliegend, das dem Schallsender gegenüberliegende Ende des Messrohrs derart ausgeformt ist, dass die von dem Schallsender erzeugte und abgestrahlte Welle reflektiert und anschließend vom Schallempfänger empfangen wird. Dies wird beispielsweise durch die Verwendung einer Reflektionsfläche erreicht.

Bevorzugt sind der Schallsender und der Schallempfänger einander gegenüberliegend angeordnet.

Noch mehr bevorzugt sind der Schallsender und der Schallempfänger derart angeordnet, dass die Schallmessstrecke entlang der Strömungsrichtung des Atemgases im Messrohr verläuft. Dies kann auf direktem Weg, beispielsweise parallel zur Strömungsrichtung, erfolgen, als auch indirekt, beispielsweise mit ein- oder mehrmaligem Durchkreuzen der Strömung des Atemgases. Die Schallmessstrecke wird dabei insgesamt zweimal in entgegengesetzter Richtung durchlaufen, d.h. einmal mit der Richtung und einmal in Gegenrichtung zur Strömung, um den Einfluss der Strömungsgeschwindigkeit auf die gemessene Schallgeschwindigkeit zu eliminieren. Insgesamt ergibt sich damit ein zweifaches Durchlaufen der Schallmessstrecke, so dass im Ergebnis der durch Überlagerung der Strömungsgeschwindkeit bewirkte Einfluss weggemittelt wird. Daraus ergibt sich, dass die Schallmessstrecke nur in geradzahliger Anzahl durchlaufen werden kann, d.h. sie kann bevorzugt zweimal, viermal, sechsmal, achtmal, zehnmal, zwanzigmal usw. durchlaufen werden.

Grundsätzlich gilt und ist aus der physikalischen Literatur belegbar, dass sich die Schallgeschwindigkeit in Gasen aus dem Druck und der Dichte nach der Beziehung

$$c_S{}^2 = \kappa \frac{p}{\rho}$$

berechnen lässt, wobei $\kappa$ der Adiabatische Koeffizient ist, der sich aus dem Quotient der spezifischen Wärme $C_p$ und $C_v$ ermittelt und p der Druck und p die Dichte darstellt. Durch Umformungen, in denen die Dichte durch die Gesamtmasse m dividiert und durch das Gesamtvolumen V ersetzt wird, und unter Anwendung der idealen Gasgleichung

$$pV = nRT$$

erhält man

$$c_S{}^2 = \kappa \frac{RT}{M}$$

wobei M die Molmasse, m die Gesamtmasse und n die Anzahl der Mole ist. Durch Messung der entsprechenden Parameter ermittelt man die Molmasse, wobei die Molmasse zur Identifizierung des entsprechenden Gases dient. Die Gesamtmasse ist hingegen unbeachtlich.

Die soeben abgeleiteten Beziehungen treffen zu bei der Bestimmung einkomponentiger Gase. Das Atemgas enthält eine Vielzahl von Gaskomponenten, sodass die gemessene Molmasse ein Wert darstellt, indem die einzelnen Gaskomponenten gewichtet mit ihren entsprechenden Anteilen im Gasgemisch zur Bildung eines Mittelwertes der Molmasse beitragen. Vergleicht man die Anteile der einzelnen Gaskomponenten im Einatem- bzw. Umgebungsgas mit dem Ausatemgas, so bleiben diese konstant mit der einzigen Ausnahme, dass das Ausatemgas einen höheren $CO_2$ und einen niedrigen $O_2$ Anteil als das Einatem- bzw. Umgebungsgas aufweist.

Über die Schallmessstrecke wird die Schalllaufzeit gemessen, vorzugsweise mittels elektronischer Mittel. Aus der Schalllaufzeit kann die Strömungsgeschwindigkeit des Atemgases und/oder die mittlere molare Masse des Atemgases bestimmt werden. Die Strömungsgeschwindigkeit (c) wird bevorzugt mittels der bereits bekannten Formel berechnet:

$$c = a * \frac{t_1 - t_2}{t_1 * t_2}$$

wobei c die Strömungsgeschwindigkeit, a eine dimensionsbehaftete Konstante und $t_1$ und $t_2$ die über die Schallmessstrecke gemessenen Schalllaufzeiten bezeichnen.

Die Schalllaufzeit ist eine Kenngröße, welche in Abhängigkeit von den Umgebungsparametern proportional zur mittleren molaren Masse des Atemgases ist. Die mittlere molare Masse des Atemgases ($M_{Atemgas}$) wird anhand der Formel berechnet:

$$M_{Atemgas} = b * T * \left(\frac{t_1 * t_2}{t_1 + t_2}\right)^2$$

wobei $M_{Atemgas}$ die mittlere molare Masse des Atemgases, T die bestimmte Temperatur des Atemgases, b eine dimensionsbehaftete Konstante und $t_1$ und $t_2$ die über die Schallmessstrecke gemessenen Schalllaufzeiten bezeichnen.

Im Rahmen der Erfindung ist die Bestimmung des Kohlenstoffdioxidanteils des Atemgases mit einem beliebigen Kohlenstoffdioxidgassensor durchführbar.
Gängig ist jedoch die Bestimmung mittels Infrarot, d.h. dass für die Bestimmung ein Infrarotsender und ein Infrarotempfänger notwendig sind. Bevorzugt ist der Kohlenstoffdioxidgassensor daher ein Infrarotempfänger, welcher ein von einem Infrarotsender gesendetes Infrarotsignal im Absorptionsbereich von Kohlenstoffdioxid empfängt. Der Kohlenstoffdioxidanteil des Atemgases wird dann aus dem von dem Infrarotempfänger über die Infrarotmessstrecke empfangenen Infrarotsignal bestimmt.

Der Begriff "Infrarotsender" ist bekannt und ist eine Quelle zur Erzeugung und Abstrahlung elektromagnetischer Wellen im Spektralbereich zwischen sichtbarem Licht und der längerwelligen Terahertzstrahlung (1 mm und 780 nm). Gleiches gilt für den Begriff "Infrarotempfänger", der ein optischer Sensor für den oben genannten Wellenlängenbereich ist. Einem Fachmann sind der Aufbau und die Funktionsweise eines Infrarotsenders und eines Infrarotempfängers bekannt.

Bevorzugt sind der Infrarotsender und der Infrarotempfänger an bzw. in dem Messrohr unlösbar integriert, so dass diese mit dem Messrohr ein einstückiges Teil bilden. Diese sind dann beispielsweise in entsprechende Ansätze des Messrohrs eingegossen. Auf diese Weise lässt sich das Messrohr mit dem Infrarotsender und dem Infrarotempfänger problemlos austauschen. Bevorzugt sind der Infrarotsender und der Infrarotempfänger an bzw. mit dem Messrohr lösbar verbunden, so dass diese und das Messrohr leicht voneinander getrennt werden können. Auf diese Weise lassen sich das Messrohr und der Infrarotsender und der Infrarotempfänger unabhängig voneinander austauschen. Weiterhin bevorzugt umfasst der Infrarotsender und der Infrarotempfänger eine Vorverstärkerelektronik, welche unlösbar in bzw. an dem Messrohr integriert oder lösbar mit diesem verbunden ist. Ein Fachmann kennt verschiedene Möglichkeiten, um dies zu realisieren.

Der Infrarotsender und der Infrarotempfänger definieren eine Infrarotmessstrecke bekannter Länge, welche bevorzugt im bzw. durch das Messrohr verläuft. Der Infrarotsender und der Infrarotempfänger können dabei jede Position zueinander einnehmen, beispielsweise gegenüberliegend und/oder nebeneinander. Es versteht sich, dass, beispielsweise im Fall der Anordnung nebeneinanderliegend, das dem Infrarotsender gegenüberliegende Ende des Messrohrs derart ausgeformt ist, dass das von dem Infrarotsender erzeugte und abgestrahlte Licht reflektiert und anschließend vom Infrarotempfänger empfangen wird. Dies wird beispielsweise durch die Verwendung eines Spiegels erreicht. Bevorzugt sind der Infrarotsender und der Infrarotempfänger einander gegenüberliegend anzuordnen.

Noch mehr bevorzugt sind der Infrarotsender und der Infrarotempfänger derart angeordnet sind, dass die Infrarotmessstrecke die Strömungsrichtung des Atemgases im Messrohr kreuzt. Bei einer Anordnung außerhalb des Messrohrs ist wenigstens ein optisch durchlässiger Bereich an entsprechender Stelle für den Durchtritt des Infrarotsignals vorgesehen. Bevorzugt ist der optisch durchlässige Bereich ein Kristallfenster. Erforderlich ist, dass das Kristallfenster in bzw. an dem Messrohr dieses gasdicht abschließt.

Die Bestimmung des Kohlenstoffdioxidanteils in dem Atemgas basiert darauf, dass Kohlenstoffdioxidmoleküle die auftreffenden infraroten Lichtwellen absorbieren, deren Frequenz im Absorptionsspektrum von Kohlenstoffdioxid liegt. Für die Messung werden über eine Infrarotmessstrecke ein Infrarotsender und ein Infrarotempfänger einander gegenüber aufgestellt. Gleichzeitig definieren der Infrarotsender und der Infrarotempfänger die Infrarotmessstrecke. Die Lichtwellen des Infrarotsenders regen die Kohlendioxidmoleküle zu Schwingungen an. Zeitverzögert fällt das Kohlenstoffdioxidmolekül in seinen unangeregten Ursprungszustand zurück und gibt die aufgenommene Energie wiederum in Form von konzentrischer Strahlung infrarotem Lichtes ab. Da nur ein geringer Teil des von dem Kohlenstoffdioxidmoleküls emittierten Infrarotlichtes sich in die gleiche Richtung ausbreitet wie das Infrarotlicht des Infrarotsenders, wird bei Anwesenheit von Kohlenstoffdioxidmolekülen in der Infrarotmessstrecke die Intensität des auf dem Infrarotempfänger auftreffenden Infrarotlichtes messbar geschwächt. Zwar treffen ebenfalls Streueffekte an Aerosolen und Luftmolekülen auf, diese sind aber für die Messung vernachlässigbar.

Das Maß der Schwächung der Intensität des vom Infrarotsender ausgestrahlten Infrarotsignals bzw. Infrarotlichtes steht somit in direktem proportionalem Zusammenhang mit der Menge der Kohlenstoffdioxidmoleküle innerhalb der Infrarotmessstrecke. D.h., je größer der Anteil an Kohlenstoffdioxid in dem Atemgas ist, desto weniger Infrarotstrahlung erreicht den Infrarotempfänger. Je kleiner der Anteil an Kohlenstoffdioxid in dem Atemgas ist, desto mehr Infrarotstrahlung erreicht den Infrarotempfänger. Befindet sich hingegen gar keine Kohlenstoffdioxidmoleküle in dem Atemgas, so gelangt die vom Infrarotsender gesendete Infrarotstrahlung vollständig zum Infrarotempfänger.

Der Begriff "Einrichtungen zur Messung der Temperatur des Atemgases" betrifft ein Messgerät zur Bestimmung der Temperatur, wie beispielsweise ein Thermometer oder Thermoelement. Im Rahmen der vorliegenden Erfindung kann die Temperatur auch elektrisch gemessen werden.

Eine derartige Einrichtung ist unlösbar in bzw. an dem Messrohr integriert oder lösbar mit diesem verbunden. Ein Fachmann kennt verschiedene Möglichkeiten, um dies zu realisieren. Zusätzlich kann eine Vorverstärkerelektronik umfasst sein.

Der Begriff "Einrichtungen zur Messung des Luftdruckes des Atemgases" ist dem Fachmann bekannt und betrifft eine Messeinrichtung zur Erfassung und/oder zum Anzeigen des physikalischen Druckes des Atemgases. Im Rahmen der vorliegenden Erfindung kann jeder beliebige Drucksensor verwendet werden, bevorzugt einer, welcher die physikalische Größe Druck in eine elektrische Ausgangsgröße als Maß für den Druck umformt.

Der Drucksensor ist unlösbar in bzw. an dem Messrohr integriert oder lösbar mit diesem verbunden. Der Fachmann kennt verschiedene Möglichkeiten, um dies zu realisieren. Es kann auch eine Vorverstärkerelektronik umfasst sein.

Der Begriff "Einrichtungen zur Messung der Feuchtigkeit des Atemgases" betrifft eine Einrichtung zur Messung der Luftfeuchtigkeit, wie beispielsweise ein Hygrometer. Im Rahmen der vorliegenden Erfindung kann die Feuchtigkeit auch elektrisch gemessen werden, beispielsweise indem ein Feuchtigkeitssensor ein elektrisches Signal liefert.

Eine derartige Einrichtung ist unlösbar in bzw. an dem Messrohr integriert oder lösbar mit diesem verbunden. Ein Fachmann kennt verschiedene Möglichkeiten, um dies zu realisieren. Zusätzlich kann eine Vorverstärkerelektronik umfasst sein.

Der Begriff "Bestimmung des Anteils an molekularem Sauerstoff in Atemgasen" betrifft die Bestimmung des Anteils oder der Konzentration an molekularem Sauerstoff in Atemgasen. Die Bestimmung erfolgt indirekt, bevorzugt mittels Differenzbildung aus der bestimmten mittleren molaren Masse des Atemgases und aus dem bestimmten Kohlenstoffdioxidanteil des Atemgases.

Die Differenzbildung basiert darauf, dass die mittlere molare Masse des Atemgases und der Kohlenstoffdioxidgehalt wenigstens einmal bestimmt werden, nämlich in dem Einatemgas bzw. in dem Umgebungsgas und/oder in dem Ausatemgas. Die im Einatemgas bzw. in dem Umgebungsgas gemessenen Werte können zur Kalibrierung des Verfahrens bzw. zum Nullpunktabgleich verwendet werden, so dass aus der Differenz der bestimmten mittleren molaren Masse des Ausatemgases und aus dem bestimmten Kohlenstoffdioxidanteil des Ausatemgases direkt der Anteil an molekularem Sauerstoff im Ausatemgas bestimmt werden kann.

Die im Einatemgas bzw. in dem Umgebungsgas gemessenen Werte werden bei jeder Bestimmung des Anteils an molekularem Sauerstoff zur Kalibrierung des Verfahrens bzw. zum Nullpunktabgleich verwendet, so dass mittels Differenzbildung direkt der Anteil an molekularem Sauerstoff im Ausatemgas bestimmt werden kann. Dies bedeutet, dass unmittelbar vor jeder Bestimmung die im Einatemgas bzw. in dem Umgebungsgas gemessenen Werte zur Kalibrierung des Verfahrens bzw. zum Nullpunktabgleich verwendet werden, bevor anschließend die Bestimmung des Anteils an molekularem Sauerstoff in dem entsprechenden Ausatemgas erfolgt.

Alternativ werden die im Einatemgas bzw. in dem Umgebungsgas gemessenen Werte nicht bei jeder Bestimmung des Anteils an molekularem Sauerstoff zur Kalibrierung des Verfahrens bzw. zum Nullpunktabgleich verwendet. Das bedeutet, dass unmittelbar vor der ersten Bestimmung die im Einatemgas bzw. in dem Umgebungsgas gemessenen Werte zur Kalibrierung des Verfahrens bzw. zum Nullpunktabgleich verwendet werden, bevor anschließend nur noch die Bestimmung des Anteils an molekularem Sauerstoff in dem Ausatemgas mittels Differenzbildung und ohne weitere Kalibrierung bzw. Nullpunktverschiebung erfolgt.

Die Bestimmung mittels Differenzbildung ist möglich, da sich die absoluten Mengen der einzelnen Komponenten oder Elemente des Atemgases, wie Stickstoff, Argon und diverse Spurengase, im Ausatemgas im Vergleich zum Einatemgas bzw. Umgebungsgas während der Messung nicht ändern. Für das Verfahren wird daher vereinfachend angenommen, dass deren Konzentrationen konstant sind. Somit kann der Einfluss dieser Komponenten oder Elemente des Atemgases bei der Messung in einer dimensionsbehafteten Konstante zusammengefasst werden.

Zudem wird für das Verfahren angenommen, dass weitere Einflüsse, wie die Strömungsgeschwindigkeit und Feuchtigkeit des Atemgases, für die Bestimmung des Anteils an molekularem Sauerstoff im Ausatemgas aus den im Folgenden genannten Gründen unberücksichtigt bleiben können:

In den Überlegungen der Konstanz der Werte wird davon ausgegangen, dass der Wert der Feuchtigkeit im Ausatemgas konstant ist und annähernd 100% beträgt. Im Normalfall ist der Einfluss der Feuchtigkeit daher vernachlässigbar. Für diese Annahme wird bevorzugt davon ausgegangen, dass eine eventuelle Zuleitung zum Messrohr eine bestimmte Länge nicht überschreitet, um eine nennenswerte Abkühlung mit Verschiebung des Taupunkts und demzufolge ein Ausfallen der Feuchtigkeit zu vermeiden. Zudem können aber Fälle auftreten, in denen eine Feuchtigkeitsmessung angezeigt ist, beispielsweise bei einer langen Zuleitung zum Messrohr und/oder einem großen Temperaturunterschied zwischen dem Einatem- bzw. Umgebungsgas und dem Ausatemgas, so dass es aufgrund der nennenswerten Abkühlung zu einer Verschiebung des Taupunkts und demzufolge zu einem Ausfallen der Feuchtigkeit kommt.

Der Einfluss der Strömungsgeschwindigkeit wird dadurch eliminiert, dass die Schallmessstrecke insgesamt zweimal in zueinander entgegengesetzter Richtung durchlaufen wird.

Somit ergibt sich ein direkter Zusammenhang zwischen dem Anteil an molekularem Sauerstoff auf der einen Seite und der Differenz der mittleren molaren Masse des Atemgases und der Konzentration an Kohlensoffdioxid auf der anderen Seite. Daraus ergibt sich vorteilhafterweise, dass die Bestimmung des Anteils an molekularem Sauerstoff in kürzester Zeit und ohne komplizierte Berechnung erfolgt. Die Bestimmung des Anteils an molekularem Sauerstoff erfolgt bevorzugt anhand bekannter mathematischer Verfahren oder, besonders bevorzugt, aus der empirisch ermittelten Abhängigkeit der Differenz der gemessenen elektrischen Signale. Erfindungsgemäß wird der Anteil an molekularem Sauerstoff ($c_{M\,O2}$) mittels der folgenden Formel berechnet:

$$c_{M\,O_2} = k_1 \left( M_{Atemgas} - k_2 * c_{M\,CO_2} - k_3 \right)$$

wobei $c_{M\,O2}$ den Anteil an molekularem Sauerstoff im Atemgas, $M_{Atemgas}$ die mittlere molare Masse des Atemgases, $c_{M\,CO2}$ den Kohlenstoffdioxidanteil im Atemgas und $k_1$, $k_2$ und $k_3$ dimensionsbehaftete Konstanten bezeichnen.

Die Messung des Einatemgases bzw. des Umgebungsgases erfolgt unmittelbar vor der Messung des Ausatemgases.

Bevorzugt wird zur Erhöhung der Genauigkeit der Bestimmung des Anteils an molekularem Sauerstoff zusätzlich die Temperatur und/oder die Feuchtigkeit des Atemgases gemessen. Noch mehr bevorzugt wird die Temperatur des Atemgases gemessen. Die Messung erfolgt mittels der oben beschriebenen Einrichtungen zur Messung.

Aufgrund dieser zusätzlichen Messung bzw. Messungen ist es möglich, die Genauigkeit der Bestimmung zu erhöhen und auch eine Kontrolle der Bestimmung durchzuführen. Beispielsweise kann eine Kontrolle der Bestimmung sinnvoll sein, wenn sich die Temperatur und/oder die Feuchtigkeit des Einatemgases bzw. des Umgebungsgases von der des Ausatemgases unterscheidet. Beispielsweise kann bei einer langen Zuleitung zum Messrohr eine Kontrolle der Bestimmung sinnvoll sein.

Es wird davon ausgegangen, dass die Definitionen und Ausführungen der oben genannten Begriffe für alle in dieser Beschreibung im Folgenden beschriebenen Aspekte gelten, sofern nichts anders angegeben ist.

Die Erfindung betrifft des Weiteren eine Vorrichtung zur Bestimmung des Anteils an molekularem Sauerstoff in einem Atemgas gemäß des Anspruchs 8 und schlägt zur Lösung der Aufgabe vor, dass die Vorrichtung durch eine Auswerteeinheit zur Bestimmung des Anteils an molekularem Sauerstoff gekennzeichnet ist, wobei die Auswerteeinheit die Differenz aus der bestimmten mittleren molaren Masse des Atemgases und aus dem bestimmten Kohlenstoffdioxidanteil ermittelt. Zu diesem Zweck werden die beiden gemessenen Signale einem Subtrahierglied zur Differenzbildung angeführt. Vorteilhafte Weiterbildungen der Erfindung, welche einzeln oder in Kombination realisierbar sind, sind in den Unteransprüchen dargestellt.

Die Vorrichtung ist vorzugsweise gasdicht ausgestaltet. Die gasdichte Ausgestaltung der Vorrichtung erstreckt sich ebenfalls auf alle von der Vorrichtung umfassten Bauteile bzw. Elemente.

Alternativ ist das verwendete Messrohr gasdicht mit der Vorrichtung verbunden. Weiterhin bevorzugt ist das Messrohr unlösbar oder lösbar mit der Vorrichtung verbunden. Das Messrohr kann im Falle der lösbaren Verbindung ausgetauscht werden.

Die Vorrichtung kann alternativ als ein Block ausgestaltet sein, welcher aus einem beliebigen Material, beispielsweise aus Metall, bestehen kann. Die Vorrichtung umfasst als Messrohr eine Bohrung. In diesem Fall sind die von dem Messrohr umfassten Bauteile bzw. Elemente in der Vorrichtung integriert.

Der Begriff "Auswerteeinheit" betrifft ein Hilfsmittel, beispielsweise einen Computer oder einen Speicherbaustein, welches in der Lage ist, die bestimmten Messwerte bzw. die gemessenen Signale aufzunehmen, zu analysieren, aufzubereiten, zu speichern und/oder weiterzuleiten. Den Input erhält das Auswertegerät durch die von der Vorrichtung umfassten Sender, Empfänger, Einrichtungen zur Messung und/oder von einer umfassten Berechnungseinheit oder auch durch Eingaben von menschlichen Nutzern.

Bevorzugt ist die Auswerteeinheit ein Computer oder ein digitaler Speicherbaustein, wie beispielsweise ein Flash-Speicher, oder ein elektronischer Speicherbaustein, wie beispielsweise ein Erasable Programmable Read-Only Memory (EPROM) oder ein Electrically Erasable Programmable Read-Only Memory (EEPROM).

Die Auswerteeinheit bestimmt den Anteil an molekularem Sauerstoff in dem Atemgas indirekt durch Subtrahieren der elektrischen Messsignale. Das Subtrahierglied ermittelt lediglich die Differenz zwischen den bestimmten Messwerten bzw. den gemessenen elektrischen Signalen, d.h. die Differenz aus der bestimmten mittleren molaren Masse des Atemgases und aus dem bestimmten Kohlenstoffdioxidanteil des Atemgases. Erfindungsgemäß ermittelt das Subtrahierglied die Differenz mittels der folgenden Formel:

$$c_{M\,O_2} = k_1 \left( M_{Atemgas} - k_2 * c_{M\,CO_2} - k_3 \right)$$

wobei $c_{M\,O2}$ den Anteil an molekularem Sauerstoff im Atemgas, $M_{Atemgas}$ die mittlere molare Masse des Atemgases, $c_{M\,CO2}$ den Kohlenstoffdioxidanteil im Atemgas und $k_1$, $k_2$ und $k_3$ dimensionsbehaftete Konstanten bezeichnen.

Die Bestimmung des Anteils an molekularem Sauerstoff erfolgt bevorzugt aus der empirisch ermittelten Abhängigkeit der ermittelten Differenz, besonders bevorzugt aus der empirisch ermittelten Abhängigkeit der ermittelten Differenz der gemessenen elektrischen Signale.

Bevorzugt umfasst die Vorrichtung zur Erhöhung der Genauigkeit der Bestimmung des Anteils an molekularem Sauerstoff zusätzlich eine Einrichtung zur Messung der Temperatur, des Luftdruckes und/oder der Feuchtigkeit des Atemgases. Noch mehr bevorzugt eine Einrichtung zur Messung der Temperatur und/oder des Luftdruckes des Atemgases Diese Einrichtungen sind ebenfalls für eine Kontrolle dieser Bestimmung geeignet. Erfindungsgemäß umfasst die Vorrichtung wenigstens einen Anschluss zur Zu- und Abführung des Atemgases, wobei dieser an das Messrohr angesetzt ist. Bevorzugt umfasst die Vorrichtung zwei Anschlüsse zur Zu- und Abführung des Atemgases. Besonders bevorzugt sind die Anschlüsse an den sich gegenüberliegenden Enden des Messrohres angesetzt, noch mehr bevorzugt sind diese senkrecht zur Achse des Messrohres angesetzt, wobei diese zueinander jedoch jeden beliebigen Winkel annehmen können.

Bevorzugt umfasst die Vorrichtung zwei verschiedene Anschlüsse zur Zu- und Abführung des Atemgases. Besonders bevorzugt strömt das über den Anschluss zur Zuführung eingeleitete Atemgas durch das Messrohr hindurch und tritt aus dem Anschluss zur Abführung wieder aus.

Weitere Einzelheiten, Merkmale und Vorteile der Erfindung ergeben sich aus der nachfolgenden Beschreibung des bevorzugten Ausführungsbeispiels in Verbindung mit den Unteransprüchen. Hierbei können die jeweiligen Merkmale für sich alleine oder zu mehreren in Kombination miteinander verwirklicht sein. Die Erfindung ist nicht auf das Ausführungsbeispiel beschränkt. Das Ausführungsbeispiel ist in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigt:

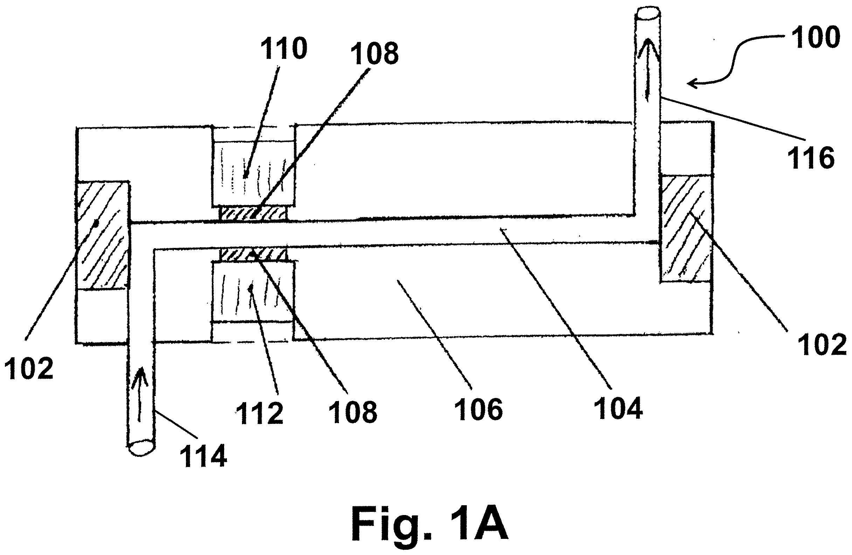

Figur 1A - 1B die erfindungsgemäße Vorrichtung in verschiedenen Ansichten.

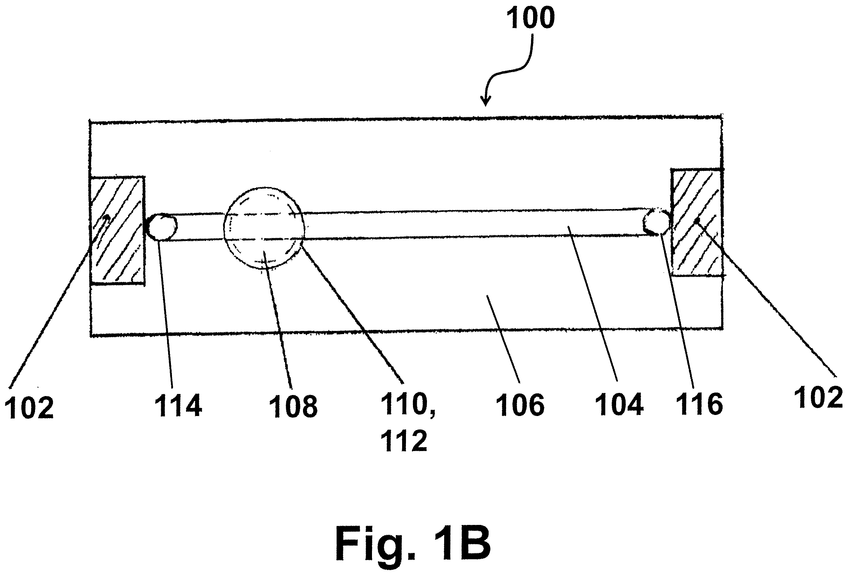

In den Figuren 1A bis 1B ist eine erfindungsgemäße Vorrichtung (100) in schematischer Darstellung in der Draufsicht (Fig. 1A) und in der Vorderansicht (Fig. 1B) gezeigt. Gleichzeitig dienen die Figuren 1A bis 1B zur Erläuterung eines möglichen Ausführungsbeispiels einer Vorrichtung zur Bestimmung des Anteils an molekularem Sauerstoff in Atemgasen.

Die Vorrichtung (100) umfasst ein Messrohr (104) in einem Metallblock (106) mit einem Schallsender (102), einem Schallempfänger (102), einem Infrarotsender (110) und einem Infrarotempfänger (112), einem optisch durchlässigem Kristallfenster (108), einer Auswerteeinheit und zwei Anschlüssen zur Zu- und Abführung des Atemgases (114, 116).

Der Schallsender (102) und der Schallempfänger (102) sind Piezoschwinger, die im Wechsel als Schallsender und Schallempfänger verwendet werden. Diese definieren eine Schallmessstrecke von bekannter Länge, welche bevorzugt im bzw. durch das Messrohr (104) verläuft. Mittels der über die Schallmessstrecke gemessenen Schalllaufzeit wird die mittlere molare Masse des Atemgases bestimmt. Der Infrarotsender (110) und der Infrarotempfänger (112) definieren eine Infrarotmessstrecke bekannter Länge, welche bevorzugt im bzw. durch das Messrohr (104) verläuft. Der Kohlenstoffdioxidanteil des Atemgases wird dann aus dem von dem Infrarotempfänger über die Infrarotmessstrecke empfangenen Infrarotsignal bestimmt.

Die Auswerteeinheit bestimmt den Anteil an molekularem Sauerstoff im Atemgas, wobei die Bestimmung mittels Differenzbildung aus der bestimmten mittleren molaren Masse des Atemgases und aus dem bestimmten Kohlenstoffdioxidanteil des Atemgases erfolgt.

Die zwei Anschlüsse zur Zu- und/oder Abführung (114, 116) sind an den sich gegenüberliegenden Enden des Messrohres (104) angesetzt. Mittels des Anschlusses zur Zuführung des Atemgases (114) gelangt dieses in die Vorrichtung, welche es mittels des Anschlusses zur Abführung (116) wieder verlässt.

**Bezugszeichenliste**

100    erfindungsgemäße Vorrichtung
102    Schallsender und/oder Schallempfänger
104    Messrohr
106    Metallblock
108    Kristallfenster
110    Infrarotsender
112    Infrarotempfänger
114    Anschluss zur Zu- und Abführung des Atemgases
116    Anschluss zur Zu- und Abführung des Atemgases

**Patentansprüche**

1. Verfahren zur synchronen Bestimmung des Anteils an molekularem Sauerstoff und Kohlendioxyd in einem Atemgas, beispielsweise in der Lungenfunktionsdiagnostik, umfassend die Schritte

- Einleiten des Atemgases in ein Messrohr (104) über ein Ende des Messrohres;
- Senden eines Schallsignals durch einen Schallsender (102) und Empfangen des Schallsignals durch einen Schallempfänger (102);
- Definieren einer Schallmessstrecke durch den Schallsender (102) und den Schallempfänger (102);
- Bestimmen der mittleren molaren Masse des Atemgases mittels der über die Schallmessstrecke gemessenen Schalllaufzeit, wobei die Schallmessstrecke insgesamt mindestens zweimal in zueinander entgegengesetzter Richtung durchlaufen wird; und
- Bestimmen des Kohlenstoffdioxidanteils des Atemgases mit einem Kohlenstoffdioxidgassensor;

**dadurch gekennzeichnet, dass**

- die Bestimmung des Anteils an molekularem Sauerstoff in dem Atemgas mittels Differenzbildung aus der bestimmten mittleren molaren Masse des Atemgases und aus dem bestimmten Kohlenstoffdioxidanteil nach der Formel

$$c_{M\,O_2} = k_1 \left( M_{Atemgas} - k_2 * c_{M\,CO_2} - k_3 \right)$$

erfolgt, wobei $c_{M\,O2}$ den Anteil an molekularem Sauerstoff im Atemgas, $M_{Atemgas}$ die mittlere molare Masse des Atemgases, $c_{M\,CO2}$ den Kohlenstoffdioxidanteil im Atemgas und $k_1$, $k_2$ und $k_3$ dimensionsbehaftete Konstanten bezeichnen, und
- die Schallmessstrecke parallel zu einer Strömungsrichtungsrichtung im Messrohr verläuft, und
- das Atemgas über das andere Ende des Messrohres abgeführt wird.

2. Verfahren nach Anspruch 1, **dadurch gekennzeichnet, dass** in einem Einatemgas bzw. Umgebungsgas gemessene Werte der mittleren Molaren Masse und des Kohlendioxid-Anteils zur Kalibrierung des Verfahrens bzw. zum Nullpunktsabgleich verwendet werden.

3. Verfahren nach einem der vorhergehenden Ansprüche, **dadurch gekennzeichnet, dass** der Schallsender (102) eine Ultraschallquelle ist und/oder der Schallempfänger (102) ein akustischer Sensor oder ein Mikrofon ist.

4. Verfahren nach einem der vorhergehenden Ansprüche, **dadurch gekennzeichnet, dass** der Kohlenstoffdioxidgassensor ein Infrarotempfänger (112) ist, welcher ein von einem Infrarotsender (110) gesendetes Infrarotsignal empfängt, wobei die Frequenz des gesendeten Infrarotsignals im Absorptionsspektrum von Kohlenstoffdioxid liegt und die Schwächung der Intensität des Infrarotsignals ein Maß für den Kohlenstoffdioxidanteil in dem Atemgas darstellt.

5. Verfahren nach dem vorhergehenden Anspruch, **dadurch gekennzeichnet, dass** eine Infrarotmessstrecke durch den Infrarotsender (110) und den Infrarotempfänger (112) definiert wird, welche die Strömungsrichtung des Atemgases im Messrohr (104) kreuzt.

6. Verfahren nach einem der Ansprüche 4 oder 5, **dadurch gekennzeichnet, dass** der Infrarotsender (110) eine Infrarotquelle ist und/oder der Infrarotempfänger (112) ein optischer Sensor ist.

7. Verfahren nach einem der vorhergehenden Ansprüche, **dadurch gekennzeichnet, dass** die Temperatur und/oder der Luftdruck des Atemgases gemessen wird.

8. Vorrichtung zur synchronen Bestimmung des Anteils an molekularem Sauerstoff und Kohlendioxyd in einem Atemgas nach dem Verfahren in Anspruch 1, beispielsweise in der Lungenfunktionsdiagnostik, mit einem Messrohr (104) umfassend einen Schallsender (102) und einen Schallempfänger (102), die eine Schallmessstrecke definieren, bei der sich mittels der über die Schallmessstrecke gemessenen Schalllaufzeit die mittlere molare Masse des Atemgases bestimmt, und einen Kohlenstoffdioxidgassensor, der den Kohlenstoffdioxidanteil des Atemgases bestimmt, wobei die Schallmessstrecke durch eine ausgesendetes Schallsignal insgesamt mindestens zweimal in entgegengesetzter Richtung durchlaufen wird,
**dadurch gekennzeichnet, dass**

- eine Auswerteeinheit vorhanden ist, welche den Anteil an molekularem Sauerstoff in dem Atemgas durch Differenzbildung mittels der folgenden Formel ermittelt:

$$c_{M\,O_2} = k_1 \left( M_{Atemgas} - k_2 * c_{M\,CO_2} - k_3 \right)$$

wobei $c_{M\,O2}$ den Anteil an molekularem Sauerstoff im Atemgas, $M_{Atemgas}$ die mittlere molare Masse des Atemgases, $c_{M\,CO2}$ den Kohlenstoffdioxidanteil im Atemgas und $k_1$, $k_2$ und $k_3$ dimensionsbehaftete Konstanten bezeichnen,
- der Schallsender (102) und der Schallempfänger (102) derart angeordnet sind, dass die Schallmessstrecke parallel zu einer Strömungsrichtung des Atemgases im Messrohr (104) verläuft, und
- die Vorrichtung wenigstens je einen Anschluss zur Zu- und Abführung (114) des Atemgases umfasst, wobei diese an sich gegenüberliegenden Enden des Messrohres (104) angesetzt sind.

9. Vorrichtung nach Anspruch 8, **dadurch gekennzeichnet, dass** der Schallsender (102) eine Ultraschallquelle ist und/oder der Schallempfänger (102) ein akustischer Sensor oder ein Mikrofon ist.

10. Vorrichtung nach einem der Ansprüche 8 oder 9, **dadurch gekennzeichnet, dass** der Schallsender (102) und der Schallempfänger (102) Piezoschwinger sind.

11. Vorrichtung nach dem vorhergehenden Anspruch, **dadurch gekennzeichnet, dass** die Piezoschwinger im Wechsel Schallsender (102) und Schallempfänger (102) sind.

12. Vorrichtung nach einem der Ansprüche 8 - 11, **dadurch gekennzeichnet, dass** ein Kohlenstoffdioxidgassensor umfassend einen Infrarotempfänger (112) vorhanden ist, welcher ein von einem Infrarotsender (110) gesendetes Infrarotsignal empfängt, wobei die Frequenz des gesendeten Infrarotsignals im Absorptionsspektrum von Kohlenstoffdioxid liegt und die Schwächung der Intensität des Infrarotsignals ein Maß für den Kohlenstoffdioxidanteil in dem Atemgas darstellt.

13. Vorrichtung nach dem vorhergehenden Anspruch, **dadurch gekennzeichnet, dass** der Infrarotsender (110) und der infrarotempfänger (112) derart angeordnet sind, dass diese eine Infrarotmessstrecke definieren, welche die Strömungsrichtung des Atemgases im Messrohr (104) kreuzt.

14. Vorrichtung nach einem der Ansprüche 12 oder 13, **dadurch gekennzeichnet, dass** der Infrarotsender (110) eine Infrarotquelle und/oder der Infrarotempfänger (112) ein optischer Sensor ist.

15. Vorrichtung nach einem der Ansprüche 8 - 14, **dadurch gekennzeichnet, dass** die Vorrichtung eine Einrichtung zur Messung der Temperatur und/oder des Luftdruckes umfasst.

**Claims**

1. Method and device for synchronous determination of the proportion of molecular oxygen in a respiratory gas, for example in lung function diagnostics, comprising the steps

- introduction of the respiratory gas into a measurement tube (104) via one end of the measurement tube;
- transmitting a sound signal by means of a sound transmitter (102) and receiving the sound signal by means of a sound receiver (102)
- defining a sound measurement section by means of the sound transmitter (102) and the sound receiver (102), determining the average molar mass of the respiratory gas by means of the sound propagation time measured over the sound measurement section, the sound measurement section being passed through a total of two times in mutually opposite directions.
- determination of the carbon dioxide proportion of the respiratory gas with a carbon dioxide gas sensor.

**characterised in that**

- the determination of the proportion of molecular oxygen in the respiratory gas is performed by means of difference formation from the determined mean molar mass of the respiratory gas and from the determined carbon dioxide proportion according to the formula

$$c_{M\,O_2} = k_1\,(M_{respiratory\,gas} - k_2 * c_{M\,CO_2} - k_3)$$

wherein $c_{M\,O2}$ is the proportion of molecular oxygen in the respiratory gas, $M_{respiratory\,gas}$ is the average molar mass of the respiratory gas , and $c_{M\,CO2}$ is the proportion of carbon dioxide in the respiratory gas and $k_1$, $k_2$ and $k_3$ are dimensioned constants, and
- the sound measurement section extends parallel to a flow direction in the measurement tube, and
- the respiratory gas is exhausted via the other end of the measurement tube.

2. Method according to Claim 1, **characterised in that,** values, measured in a respiratory gas or ambient gas, for the average molar mass and carbon dioxide proportion are used for calibrating the method or for nullification.

3. Method according to any one of the preceding claims, **characterised in that** the sound transmitter (102) is an ultrasound source and/or the sound receiver (102) is an acoustic sensor or a microphone.

4. Method according to any one of the preceding claims, **characterised in that** the carbon dioxide gas sensor is an infrared receiver (112), which receives an infrared signal transmitted by an infrared transmitter (110), the frequency of the transmitted infrared signal lying in the absorption spectrum of carbon dioxide and the attenuation of the intensity of the infrared signal representing a measure of the carbon dioxide proportion in the respiratory gas.

5. Method according to the preceding claim, **characterised in that** an infrared measurement section is defined by the infrared transmitter (110) and the infrared receiver (112), which crosses the flow direction of the respiratory gas in the measurement tube (104).

6. Method according to one claims 4 or 5, **characterised in that** the infrared transmitter (110) is an infrared source and/or the infrared receiver (112) is an optical sensor.

7. Method according to one of the preceding claims, **characterised in that** the temperature and/or the air pressure of the respiratory gas is measured.

8. Device for synchronously determining the proportion of molecular oxygen and carbon dioxide in a respiratory gas according to the method in Claim 1, for example in lung function diagnostics, comprising a measurement tube (104), which comprises a sound transmitter (102) and a sound receiver (102), which define a sound measurement section, in which the average molar mass of the respiratory gas is determined by means of the sound propagation time measured over the sound measurement section, and a carbon dioxide gas sensor, which determines the carbon dioxide proportion of the respiratory gas, the sound measurement section being passed through by a transmitted sound signal a total of two times in mutually opposite directions.
**characterised in that**

- an evaluation unit is provided, which determines the proportion of molecular oxygen in the respiratory gas by subtraction by means of the following formula:

$$c_{M\,O_2} = k_1\,(M_{respiratory\,gas} - k_2 * c_{M\,CO_2} - k_3)$$

wherein $c_{M}\,O_2$ is the proportion of molecular oxygen in the respiratory gas, $M respiratory gas$ is the average molar mass of the respiratory gas , and $c_{M\,CO2}$ is the proportion of carbon dioxide in the respiratory gas and $k_1$, $k_2$ and $k_3$ represent dimensioned constants.
- the sound transmitter (102) and the sound receiver (102) being disposed such that the sound measurement section extends parallel to a flow direction of the respiratory gas in the measurement tube (104), and
- the device comprises at least one connection for feeding and exhaust (114) of the respiratory gas, it being mounted on the opposite ends of the measurement tube (104).

9. Device according to Claim 8, **characterised in that** the sound transmitter (102) is an ultrasound source and/or the sound receiver (102) is an acoustic sensor or a microphone.

10. Device according to any one of claims 8 or 9, **characterised in that** the sound transmitter (102) and the sound receiver (102) are piezo oscillators.

11. Device according to the preceding claim, **characterised in that** the piezo oscillators are alternately sound transmitters (102) and sound receivers (102).

12. Device according to any one of claims 8 - 11, **characterised in that** a carbon dioxide gas sensor, comprising an infrared receiver (112) is provided, which receives an infrared signal transmitted by an infrared transmitter (110), the frequency of the transmitted infrared signal lying in the absorption spectrum of carbon dioxide and the attenuation of the intensity of the infrared signal representing a measure of the carbon dioxide proportion in the respiratory gas.

13. Device according to the preceding claim, **characterised in that** the infrared transmitter (110) and the sound receiver (112) are arranged such that they define an infrared measurement section, which crosses the flow direction of the respiratory gas in the measurement tube (104).

14. Device according to any one of claims 12 or 13, **characterised in that** the infrared transmitter (110) is an infrared source and/or the infrared receiver (112) is an optical sensor.

15. Device according to any one of claims 8 - 14, **characterised in that** the device comprises a means for measuring the temperature and/or the air pressure.

**Revendications**

1. Procédé destiné à la détermination synchrone de la part d'oxygène moléculaire et du dioxyde de carbone dans un masque respiratoire, par exemple dans le diagnostic de fonction pulmonaire, comprenant les étapes

   - Introduction du gaz respiratoire dans un tube de mesure (104) par une extrémité du tube de mesure ;
   - Émission d'un signal acoustique par un émetteur acoustique (102) et réception du signal acoustique par un récepteur acoustique (102) ;
   - Définition d'un tronçon de mesure de la propagation du son par l'émetteur acoustique (102) et le récepteur acoustique (102) ;
   - Détermination de la masse molaire moyenne du gaz respiratoire au moyen de la durée de propagation du son mesurée sur le tronçon de mesure de la propagation du son, sachant que le tronçon de mesure de la propagation du son est traversé en tout au moins deux fois dans des sens opposés ; et
   - Détermination de la part de dioxyde de carbone du gaz respiratoire à l'aide d'un capteur de dioxyde de carbone ;

   **caractérisé par le fait que**

   - la détermination de la part d'oxygène moléculaire dans le gaz respiratoire a lieu en soustrayant la part de dioxyde de carbone déterminée de la masse molaire moyenne déterminée du gaz respiratoire d'après la formule

$$C_{M\,O_2} = k_1\,(M_{Gaz\,respiratoire} - k_2 * C_{M\,CO_2} - k_3),$$

   sachant que $C_{M\,O_2}$ désigne la part d'oxygène moléculaire dans le gaz respiratoire, $M_{Gaz\,respiratoire}$ la masse molaire moyenne du gaz respiratoire, $C_{M\,CO_2}$ la part de dioxyde de carbone dans le gaz respiratoire et $k_1$, $k_2$ et $k_3$ des constantes liées aux dimensions,
   - le tronçon de mesure de la propagation du son suivant un parcours parallèle à un sens de l'écoulement dans le tube de mesure, et
   - le gaz respiratoire étant prélevé par l'autre extrémité du tube de mesure.

**2.** Procédé selon la revendication 1, **caractérisé par le fait que,** dans un gaz respiratoire inspiré ou un gaz ambiant, les valeurs mesurées de la masse molaire moyenne et de la part de dioxyde de carbone sont utilisées pour calibrer le procédé ou pour compenser le point zéro.

**3.** Procédé selon une des revendications précédentes, **caractérisé par le fait que** l'émetteur acoustique (102) est une source d'ultrasons et/ou le récepteur acoustique (102) un capteur acoustique ou un microphone.

**4.** Procédé selon une des revendications précédentes, **caractérisé par le fait que** le capteur de dioxyde de carbone est un récepteur d'infrarouge (112) qui reçoit un signal infrarouge émis par un émetteur infrarouge (110), sachant que la fréquence du signal infrarouge envoyé se trouve dans le spectre d'absorption du dioxyde de carbone et que l'affaiblissement de l'intensité du signal infrarouge représente une mesure de la part de dioxyde de carbone dans le gaz respiratoire.

**5.** Procédé selon la revendication précédente, **caractérisé par le fait qu**'un tronçon de mesure d'infrarouge est défini par l'émetteur d'infrarouge (110) et le récepteur d'infrarouge (112), qui croise le sens de l'écoulement du gaz respiratoire dans le tube de mesure (104).

**6.** Procédé selon une des revendications 4 ou 5, **caractérisé par le fait que** l'émetteur infrarouge (110) est une source d'infrarouges et/ou le récepteur infrarouge (112) est un capteur optique.

**7.** Procédé selon une des revendications précédentes, **caractérisé par le fait que** la température et/ou la pression de l'air du masque respiratoire est mesurée.

**8.** Dispositif destiné à déterminer de façon synchrone la part d'oxygène moléculaire et de dioxyde de carbone dans un gaz respiratoire d'après le procédé de la revendication 1, par exemple dans le diagnostic de la fonction pulmonaire, avec un tube de mesure (104) entourant un émetteur acoustique (102) et un récepteur acoustique (102), qui définissent un tronçon de mesure de la propagation du son permettant de déterminer la masse molaire moyenne du gaz respiratoire au moyen de la durée de propagation du son sur le tronçon de mesure de la propagation du son, et un capteur de dioxyde de carbone qui détermine la part de dioxyde de carbone dans le gaz respiratoire, sachant que le tronçon de mesure de la propagation du son est traversé en tout au moins deux fois dans des sens opposés, **caractérisé par le fait**

- **qu'**il existe une unité d'évaluation qui détermine la part d'oxygène moléculaire dans le gaz respiratoire par soustraction au moyen de la formule suivante :

$$C_{M\,O^2} = k_1\,(M_{Gaz\;respiratoire} - k_2 * C_{M\,CO^2} - k_3),$$

sachant que $C_{M\,O_2}$ désigne la part d'oxygène moléculaire dans le gaz respiratoire, $M_{Gaz\;respiratoire}$ la masse molaire moyenne du gaz respiratoire, $C_{M\,CO_2}$ la part de dioxyde de carbone dans le gaz respiratoire et $k_1$, $k_2$ et $k_3$) des constantes liées aux dimensions,
- **que** l'émetteur acoustique (102) et le récepteur acoustique (102) sont disposés de manière à ce que le tronçon de mesure de la propagation du son suive un parcours parallèle au sens d'écoulement du gaz respiratoire dans le tube (104), et
- **que** le dispositif entoure à chaque fois au moins un raccordement à l'amenée et à l'évacuation (114) du gaz respiratoire, sachant que ces derniers sont disposés aux extrémités opposées du tube de mesure (104).

**9.** Dispositif selon la revendication 8 ou 9, **caractérisé par le fait que** l'émetteur acoustique (102) est une source d'ultrasons et/ou le récepteur acoustique (102) est un capteur acoustique ou un microphone.

**10.** Dispositif selon la revendication 8 ou 9, **caractérisé par le fait que** de l'émetteur acoustique (102) et le récepteur acoustique (102) sont des oscillateurs piézo-électriques.

**11.** Dispositif selon une des revendications précédentes, **caractérisé par le fait que** les oscillateurs piézo-électriques sont alternativement émetteurs acoustique (102) et récepteurs acoustique (102).

**12.** Dispositif selon une des revendications 8 à 11, **caractérisé par le fait qu**'il y a un capteur de gaz de dioxyde de carbone entourant un récepteur d'infrarouge (112) qui reçoit un signal infrarouge émis par un émetteur infrarouge (110), sachant que la fréquence du signal infrarouge émis se situe dans le spectre d'absorption du dioxyde de carbone et que l'affaiblissement de l'intensité du signal infrarouge représente une mesure de la part de dioxyde de carbone dans le gaz respiratoire.

13. Dispositif selon une des revendications précédentes, **caractérisé par le fait que** l'émetteur infrarouge (110) et le récepteur infrarouge (112) sont disposés de manière à ce que ces derniers définissent un tronçon de mesure des infrarouges qui croise le sens de l'écoulement du gaz respiratoire dans le tube de mesure (104).

14. Dispositif selon une des revendications 12 ou 13, **caractérisé par le fait que** l'émetteur infrarouge (110) est une source d'infrarouges et/ou le récepteur infrarouge (112) est un capteur optique.

15. Dispositif selon une des revendications 8 à 14, **caractérisé par le fait que** le dispositif entoure un dispositif destiné à mesurer la température et/ou la pression de l'air.

**Fig. 1A**

EP 3 111 207 B1

100

102    114    108    110,    106    104    116    102
                      112

Fig. 1B

EP 3 111 207 B1

**IN DER BESCHREIBUNG AUFGEFÜHRTE DOKUMENTE**

**In der Beschreibung aufgeführte Patentdokumente**

- EP 0533980 A1
- EP 1279368 A2
- EP 0646346 A2
- EP 0653919 B1
- DE 4318690 A1
- DE 102008056279 A1